(19) Europäisches Patentamt
European Patent Office
Office européen des brevets

(11) Veröffentlichungsnummer: **0 199 276**
**A2**

(12) # EUROPÄISCHE PATENTANMELDUNG

(21) Anmeldenummer: **86105252.0**

(51) Int. Cl.⁴: **C07D 265/22**

(22) Anmeldetag: **16.04.86**

(30) Priorität: **19.04.85 DE 3514183**

(43) Veröffentlichungstag der Anmeldung:
**29.10.86 Patentblatt 86/44**

(84) Benannte Vertragsstaaten:
**BE CH DE FR GB IT LI**

(71) Anmelder: **BASF Aktiengesellschaft**
**Carl-Bosch-Strasse 38**
**D-6700 Ludwigshafen(DE)**

(72) Erfinder: **Hamprecht, Gerhard, Dr.**
**Rote-Turm-Strasse 28**
**D-6940 Weinheim(DE)**
Erfinder: **Rohr, Wolfgang, Dr.**
**In der Dreispitz 13**
**D-6706 Wachenheim(DE)**
Erfinder: **Varwig, Juergen, Dr.**
**Bitterstrasse 21**
**D-6900 Heidelberg(DE)**

(54) **Verfahren zur Herstellung substituierter 2-Phenyl-4H-3,1-benzoxazin-4-one.**

(57) Verfahren zur Herstellung von 2-Phenyl-4H-3,1-benzoxazin-4-onen der allgemeinen Formel I

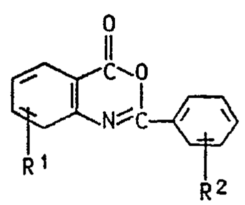

(I),

worin $R^1$ und $R^2$ Wasserstoff oder Halogen, $R^1$ ferner Methyl oder Methoxy, $R^2$ ferner Halogenalkyl, Halogenalkoxy, Halogenalkylmercapto oder Halogenalkylsulfonyl mit jeweils 1 bis 3 Kohlenstoffatomen bedeutet, durch Umsetzung einer entsprechenden Anthranilsäure mit einem entsprechenden Benzoylhalogenid in Gegenwart einer Base - (Acylierung) und Ringschluß durch Wasserentzug - (Cyclisierung), in einem mit wäßriger Alkalilauge nicht mischbaren Suspensionsmittel, in Gegenwart eines Phasentransferkatalysators.

EP 0 199 276 A2

# Verfahren zur Herstellung substituierter 2-Phenyl-4H-3,1-benzoxazin-4-one

Die Erfindung betrifft ein Verfahren zur Herstellung von substituierten 2-Phenyl-4H-3,1-benzoxazin-4-onen der allgemeinen Formel I

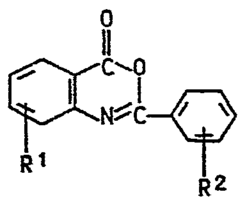

(I),

worin $R^1$ und $R^2$ Wasserstoff oder Halogen, $R^1$ ferner Methyl oder Methoxy, $R^2$ ferner Halogenalkyl, Halogenalkoxy, Halogenalkylmercapto oder Halogenalkylsulfonyl mit jeweils 1 bis 3 Kohlenstoffatomen bedeutet, durch Umsetzung entsprechend substituierter Anthranilsäure und Benzoylhalogenid in Gegenwart eines anorganischen Säureakzeptors bzw. einer Base.

Verwendet man 6-Chloranthranilsäure und als Benzoylhalogenid Benzoylchlorid als Ausgangsstoffe, so kann der Reaktionsablauf durch folgendes Formelschema wiedergegeben werden:

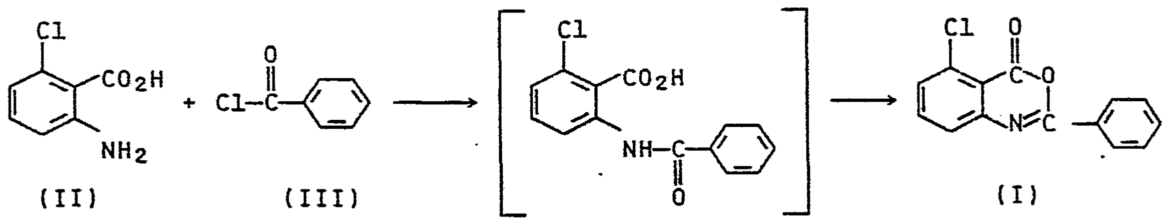

(II) (III) (I)

Man kann nach der EP-A-17 931 auch zunächst eine Anthranilsäure mit einer ungefähr stöchiometrischen Menge eines Benzoylhalogenids und einer organischen Base in einem inerten organischen Lösungsmittel zur N-Benzoylanthranilsäure umsetzen, das Zwischenprodukt durch Behandlung mit Salzsäure und durch Extraktion mit Alkali und Ansäuern reinigen, trocknen und dann mit einem billigen Säurehalogenid oder einem anderen wasserentziehenden Mittel cyclisieren. Es schließt sich eine nochmalige Wasserwäsche an. Die Gesamtausbeute beträgt je nach Substitutionsmuster der Einsatzstoffe 60 % (EP 17 931, Beispiel 2), 89 % (EP 32 242, Beispiel 2) bzw. 74 % (DE-OS 30 37 970, Beispiel 2).

Die Raum-Zeit-Ausbeute (RZA) als Maß für die Verfahrenswirtschaftlichkeit beträgt zunächst für die reine Reaktionszeit beider Stufen 0,105 kg/l/Tag (EP 17 931, Beispiel 2); wegen der erforderlichen, verschiedenen Verfahrensschritte der 1. Stufe mit einem Extraktions-, Umfäll-, Absaug-und Trocknungsprozeß sowie der Destillation des Lösungsmittels selbst neben der verlustreichen Aufarbeitung der wasserlöslichen Base erniedrigt sich jedoch noch die RZA auf ca.0,05 kg/l/Tag. Technisch nachteilig ist auch das Auftreten eines trockenen staubenden Zwischenprodukts, das sich auch wenig für die fortlaufend betriebene Verfahrensweise eignet.

In J. Org. Chem. 9, 396 (1944) wird eingehend ein Verfahren zur Benzoylierung von Anthranilsäure in verdünnter Natronlauge beschrieben. Dabei muß stark verdünnte Natronlauge -z.B. 0,2 n -verwendet werden, da bei höherer Konzentration das Natriumsalz der N-Benzoylanthranilsäure auskristallisiert und infolge von Nebenreaktionen eine insgesamt niedrigere Ausbeute erhalten wird. Die Reaktion verläuft langsam -z.B. während 3 Stunden-und bei niedriger Reaktionstemperatur unter intensivem Rühren und erfordert einen mindestens zweifachen Überschuß an Alkali. Die angegebenen Verfahrensumstände der weiterhin erforderlichen Reinigungsschritte -wobei organische Lösungsmittel auszuschließen sind-lassen diese für das Laboratorium gedachte Methode aus technischer Sicht nicht geeignet erscheinen.

Von X.Huang und Ch.-Ch.Chan ist in Synthesis 1984 (10), 851-52 ein Verfahren zur Herstellung von u.a. 3-Oxo-3,4-dihydro-2H-1,4-benzoxazinen in Gegenwart von Phasentransfer-Katalysatoren beschrieben worden. Dieses Verfahren kommt der nachstehend beschriebenen Erfindung jedoch nur scheinbar nahe, da es eine mindestens stöchiometrische Menge an Benzyltrimethylammoniumsalz benötigt.

Aufgabe der Erfindung ist die Schaffung eines einfachen, im technischen Maßstab befriedigenden Verfahrens zur Herstellung von 2-Phenyl-4H-3,1-benzoxazin-4-onen der Formel I.

Es wurde gefunden, daß ein solches Verfahren zweckmäßig darin besteht, daß man die Anthranilsäure in einem organischen, mit wäßriger Alkalilauge nicht mischbaren Lösungsmittel vorlegt und mit jeweils stöchiometrischen Mengen mindestens 25 %iger Alkalilauge einerseits und Benzoylhalogenid andererseits gleichzeitig oder nacheinander in Gegenwart eines Phasentransferkatalysators versetzt (Acylierung) und die gebildete N-Benzoylanthranilsäure nach Entfernung des Wassers in an sich bekannter Weise cyclisiert. Es versteht sich, daß man anstelle der Anthranilsäure auch deren Alkalisalz verwenden kann; in diesem Fall entfällt der Zusatz an Alkalilauge.

Im Hinblick auf den Stand der Technik liefert das erfindungsgemäße Verfahren auf einfacherem und wirtschaftlicherem Wege 2-Phenyl-4H-3,1-benzoxazin-4-one in vergleichsweise besserer Ausbeute und Reinheit und ist auch in mehrfacher Hinsicht überraschend:

Wie eingangs erwähnt, war bei Verwendung konzentrierter Natronlauge eine verringerte Ausbeute durch Auskristallisieren des Zwischenproduktes und das Auftreten von Nebenreaktionen (z.B. Hydrolyse von Benzoylhalogenid) zu erwarten und es sollte die Verwendung eines organischen Lösungsmittels vermieden werden.

Wie der nachstehende Vergleichsversuch lehrt, führt auch die Substitution der Anthranilsäure, z.B. durch Halogen in 6-Stellung, zu stark herabgesetzter Reaktionsgeschwindigkeit, so daß bei der in J. Org. Chem. beschriebenen Methode das Zwischenprodukt nur mit 71,5 % Ausbeute erhalten wird.

Schließlich ist allgemein bekannt, wie schon angedeutet, daß Benzoylhalogenide, die an sich in Wasser schlecht löslich sind, bei löslichkeitsvermittelnden Zusätzen (organische Lösungsmittel, z.B. Ether) schnell hydrolysieren, besonders dann, wenn statt Wasser wäßriges Alkali zugegen ist.

Entgegen der Erwartung reagiert jedoch bei erfindungsgemäßer Verfahrensweise das Benzoylhalogenid ausschließlich mit der Aminogruppe der Anthranilsäure.

Entgegen der Erwartung muß ferner die Reaktion auch weder bei niedriger Temperatur und entsprechendem Aufwand an Kühlenergie noch mit besonderem Rühraufwand o.ä. durchgeführt werden. Schließlich liegt die Raum-Zeit-Ausbeute des erfindungsgemäßen Verfahrens um ein Mehrfaches über der des bekannten.

Bevorzugte Ausgangsstoffe II und III und dementsprechend bevorzugte Zielprodukte I sind solche, in deren Formeln $R^1$ und $R^2$ Wasserstoff oder Halogen bedeuten. Die Ausgangsstoffe II und III sollten aus wirtschaftlichen Gründen in stöchiometrischer Menge angewendet werden; ein Überschuß der einen Komponente über die andere, z.B. in einem Verhältnis von 0,9 bis 1,1, insbesondere 1 bis 1,05 mol Ausgangsstoff III je Mol Ausgangsstoff II ist jedoch technisch ohne Nachteil.

Beispielsweise kommen folgende 2-Aminobenzoesäuren (II) in Betracht: Anthranilsäure, 6-Chlor-, 6-Fluor-, 6-Brom-anthranilsäure; 6-Methyl-, 6-Methoxy-anthranilsäure, 5-Fluor-, 5-Chlor-, 5-Brom-, 5-Methyl-, 5-Methoxy-anthranilsäure, 4-Fluor-, 4-Chlor-, 4-Brom-, 4-Methyl-, 4-Methoxy-anthranilsäure, 3-Fluor-, 3-Chlor-, 3-Brom-, 3-Methyl-, 3-Methoxy-anthranilsäure.

Benzoylhalogenide (III) sind beispielsweise: Benzoylchlorid oder -bromid; m, p-Fluor-benzoylchlorid, m, p-Chlorbenzoylchlorid, m, p-Triflourmethoxy-benzoylfluorid, m, p-Chlordifluormethoxy-benzoylfluorid, m, p-Difluormethoxy-benzoylchlorid, m, p-Trifluormethylmercapto-benzoylfluorid, m, p-Chlordifluormethylmercapto-benzoylfluorid, m, p-Difluormethylmercapto-benzoylchlorid, m, p-1,1,2,2-Tetrafluorethoxy-benzoylchlorid, m, p-1,1,2-Trifluor-2-chlorethoxy-benzoylchlorid, m, p-1,1,2,2-Tetrafluorethylmercapto-benzoylchlorid, m, p-Chlormethylsulfonyl-benzoylchlorid, m, p-Trifluormethylsulfonyl-benzoylfluorid.

Die Acylierung wird bei einer Temperatur von 10 bis 50°C, vorzugsweise 20 bis 45°C vorgenommen, wobei alle üblichen Maßnahmen der Verfahrenstechnik, z.B. fortlaufender oder absätzweiser Betrieb, Rührkessel, Rohrreaktoren usw. angewendet werden können.

Man legt eine vorgewählte Menge an Anthranilsäure oder deren Alkalisalz in einem unter den Reaktionsbedingungen inerten, mit Wasser nicht mischbaren organischen Suspensionsmittel in Anwesenheit eines Phasentransferkatalysators vor. Als organische Suspensionsmittel eignen sich besonders chlorierte Kohlenwasserstoffe wie Tetrachlorethylen, 1,1,2,2-oder 1,1,1,2-Tetrachlorethan, Dichlorpropan, Methylenchlorid, Dichlorbutan; Chloroform, Chlornaphthalin, Dichlornaphthalin, Tetrachlorkohlenstoff, 1,1,1-oder 1,1,2-Trichlorethan, Trichlorethylen, Pentachlorethan, o-, m-, p-Difluorbenzol, 1,2-Dichlorethan, 1,1-Dichlorethan, 1,2-Dichlorethylen; Chlorbenzol, Fluorbenzol, Brombenzol, Jodbenzol, o-, p-und m-Dichlorbenzol, o-, p-, m-Dibrombenzol, o-, m-, p-Chlortoluol, 1,2,4-Trichlorbenzol; Ether, soweit sie nicht zu sehr wasserlöslich sind, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Thioanisol, Nitrokohlenwasserstoffe wie Nitromethan, Nitroethan, Nitrobenzol, o-, m-, p-Chlornitrobenzol, o-Nitrotoluol; Kohlenwasserstoffe, z.B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Toluol, Xylol und entsprechende Gemische; Ester, z.B. Ethylacetat, Acetessigester, Isobutylacetat; Amide, z.B. 2-Ethylhexansäuredimethylamid. Zweckmäßig verwendet man das Lösungsmittel in einer Menge von 1000 bis 3000 Gew.%, vorzugsweise 1200 bis 1800 Gew.%, bezogen auf Anthranilsäure; ein löslichkeitsbedingter Wasseranteil von z.B. bis zu 5% oder etwas mehr ist ohne Nachteil.

Falls man nicht von vornherein das Alkalisalz der Anthranilsäure verwendet, muß nunmehr Alkalilauge zugesetzt werden; dies kann gleichzeitig mit dem Benzoylhalogenid oder vorher geschehen.

Als Alkalilauge kommen mindestens 25 % wäßrige Lösungen von Natriumhydroxid oder Kaliumhydroxid in Frage, die in annähernd stöchiometrischer Menge, z.B. in einem Verhältnis von 0,9 bis 1,1, insbesondere 1 bis 1,05 mol je mol Ausgangsstoff II eingesetzt werden. Zwar kann man auch in praktisch wasserfreien Systemen arbeiten, jedoch erzielt man eine höhere Reinheit, wenn man wäßrige Lauge verwendet.

Als Säurehalogenide können bei der Cyclisierung Thionylchlorid, Phosgen, Phosphortrichlorid, Phosphorpentachlorid oder Phosphoroxychlorid eingesetzt werden. Die Menge des Säurechlorids beträgt zweckmäßig 0,9 bis 1,3 mol, vorzugsweise 0,95 bis 1,15 mol pro Mol Ausgangsstoff II im Falle der Verwendung von Thionylchlorid oder Phosgen, sowie 0,6 bis 1 Mol im Falle der Verwendung von Phosphortrichlorid, Phosphoroxychlorid, Phosphorpentachlorid.

Als Phasentransferkatalysator eignen sich quartäre Ammoniumsalze wie beispielsweise Benzyltrimethylammoniumchlorid, Benzyltriethylammoniumchlorid, Benzyltri-n-butylammoniumchlorid, Benzyltrimethylammoniumhydroxid, Tetraethylammoniumchlorid, Tetraethylammoniumbromid, Tetrabutylammoniumjodid, Cetyltrimethylammoniumbromid, Methyltributylammoniumjodid, Myristyltrimethylammoniumbromid, Phenyltrimethylammoniumjodid, Tetramethylammonium-tetrafluorborat und quartäre Phosphoniumsalze wie beispielsweise Benzyltriphenylphosphoniumchlorid, Hexadecyltributylphosphoniumbromid, Methyltriphenylphosphoniumbromid, Methyltrioctylphosphoniumchlorid, Dodecyltriphenylphosphoniumbromid, n-Propyltriphenylphosphoniumbromid.

Der Katalysator wird in katalytisch wirksamer Menge, z.B. 0,1 bis 5 Gew.%, vorteilhaft 1 bis 2 Gew.%, bezogen auf den Rohstoff II - (Aminobenzoesäure) verwendet.

Vorteilhaft wird das Verfahren so durchgeführt, daß man über zwei Zuführungen das Benzoylhalogenid und die äquivalente Menge an wäßriger Lauge innerhalb von 10 bis 80 Minuten bei 10 bis 50°C, vorzugsweise 20 bis 45°C zur Anthranilsäure, die sich teils gelöst, überwiegend aber suspendiert in dem Suspensionsmittel befindet, zulaufen läßt. Dabei kann die Lauge jeweils mit einem zeitlichen Vorsprung oder überhaupt von vornherein zugesetzt werden.

Anschließend entfernt man auf übliche Weise das Wasser aus dem Reaktionsgemisch; wenn das Suspensionsmittel geeignet ist, eine Azeotropdestillation durchzuführen, so entfernt man das Wasser azeotrop aus dem Reaktionsgemisch. Bei höhersiedenden Suspensionsmitteln kann man dies auch unter vermindertem Druck.

Nunmehr nimmt man den zweiten Reaktionsschritt vor, die Cyclisierung unter Wasseraustritt, wobei man als wasserbindendes Mittel zweckmäßig ein preiswertes Säurehalogenid verwendet. In diesem Falle benötigt man eine Temperatur von zunächst 20 bis 80°C und rührt dann bei bis zu 140°C, vorzugsweise 50 bis 110°C bis zum Ende der Gasabspaltung während 1 bis 3 Stunden nach. Falls ein gasförmiges Säurehalogenid, z.B. Phosgen, gewählt wurde, kann man auch gleich bei 50 bis 110°C arbeiten. Zweckmäßig gibt man bei Verwendung von Phosgen als Katalysator ein Säureamid vom Typ des N,N-Dimethylformamids in einer Menge von 0,2 bis 3 Gew.%, vorteilhaft 0,5 bis 1 Gew.% (bezogen auf Anthranilsäure) hinzu. Bei dieser Wasserabspaltung geht der Säureamidcharakter des Zwischenprodukts verloren und es tritt, wenn ein geeignetes Suspensionsmittel verwendet wurde, Lösung ein.

Zur Gewinnung des Zielprodukts saugt man das in dem Reaktionsgemisch ausgefallene Neutralsalz (i.a. Kochsalz) und etwaige unlösliche Verunreinigungen ab und engt das Filtrat ein. Das so gewonnene Endprodukt ist bereits genügend rein, um z.B. als Herbizid eingesetzt oder für Folgereaktionen verwendet werden zu können; man kann es natürlich bei Bedarf weiter reinigen, z.B. durch Destillation. Man kann auch so vorgehen, daß man auf ca. 30 bis 50°C abkühlt, Reste überschüssigen Säurechlorids durch Zusatz von Wasser zersetzt und anschließend mit Wasser oder verdünntem wäßrigen Alkali extrahiert. Das lösungsmittelhaltige Filtrat wird wiederum eingeengt.

Die Zielprodukte sind wertvolle, selektiv wirkende Herbizide (vgl. DE-OS 29 14 915) oder können für die Herstellung von weiterentwickelten Herbiziden verwendet werden.

Beispiel 1

70,3 g Benzoylchlorid und 40 g 50 %ige wäßrige Natronlauge werden innerhalb von 40 min bei 23 bis 42°C unter Rühren gleichzeitig über 2 Zulauf vorrichtungen zu einer Mischung von 85,8 g feinteiliger 6-Chloranthranilsäure, 0,9 g Trimethylbenzylammoniumchlorid und 1250 g 1,2-Dichlorethan zugesetzt. Man rührt noch 90 min bei 28°C, kreist das Wasser während 1 Stunde unter Rückfluß aus und setzt bei 70°C innerhalb von 45 min unter Rühren 63,1 Teile Thionylchlorid zu. Nach weiterem Rühren während 90 min bei 82°C wird auf 40°C abgekühlt und 3mal mit Wasser extrahiert. Nach dem Einengen i.V. zuletzt bei 140°C/20 mbar erhält man 126,2 g (=98 %) 5-Chlor-2-phenyl-4H-3,1-benzoxazin-4-on (Fp.151 bis 153°C). Nach HPLC-Vergleich mit einem gereinigten Standard beträgt die Reinheit 96 %.

Beispiel 2

70,3 g Benzoylchlorid und 40 g 50 %ige wäßrige Natronlauge werden innerhalb von 40 min bei 20 bis 35°C unter Rühren gleichzeitig über 2 Zulaufvorrichtungen zu einer Mischung von 85,8 g 6-Chloranthranilsäure, 1,3 g Tetrabutylammoniumjodid, 0,5 g Dimethylformamid und 1300 g 1,2-Dichlorbenzol zugesetzt. Man rührt noch 90 min bei 30°C, kreist das Wasser bei 75 bis 90°C/20 bis 50 mbar aus und setzt über ein Gaseinleitungsrohr 64 g Phosgen während 1 Stunde bei 100 bis 105°C unter Rühren zu. Man läßt auf Raumtemperatur unter Durchleiten von Stickstoff abkühlen, filtriert vom Ungelösten ab und engt ein, zuletzt bei 140°C/20 mbar. Nach der Destillation bei 221 bis 230°C/4 bis 5 mbar erhält man 121 g (=94 %) 5-Chlor-2-phenyl-4H-3,1-benzoxazin-4-on (Fp.155 bis 157°C; HPLC-Reinheit 99,5 %).

Vergleichsversuch

Nach dem in J. Org. Chem. 9, 396 (1944) beschriebenen Verfahren läßt man bei 1°C unter intensivem Rühren 28,1 g Benzoylchlorid und 100 ml 0,2 n Natronlauge zu einer Lösung von 34,3 g 6-Chloranthranilsäure in 1 l 0,2 n Natronlauge innerhalb von 60 min zulaufen. Der ausgefallene Niederschlag löst sich auch nach dem Ansteigen der Temperatur auf 25°C nicht und wird nach weiteren 60 min Rührens abgesaugt und getrocknet, wobei 8,3 g Rückstand anfallen. Das Filtrat wird in einem 2 l-Becherglas tropfenweise mit 5 n Salzsäure versetzt, bis Kristallisation einsetzt. Man erwärmt nun auf 70°C und versetzt unter intensivem Rühren mit insgesamt 40 ml 5 n Salzsäure. Anschließend wird in einem Eisbad abgekühlt, mit weiteren 10 ml 5 n Salzsäure versetzt und abgesaugt; die Kristalle werden portionsweise mit insgesamt 1 l eiskaltem Wasser gewaschen, abgesaugt und über Nacht getrocknet. Es werden 32,7 g N-Benzoyl-6-chloranthranilsäure vom Fp. 208 bis 210°C erhalten. Der aus der Reaktion angefallene Rückstand kann erst nach Zugabe von 1,4 g Natriumhydroxid in 200 ml Wasser unter Rühren größtenteils gelöst werden. Nach Abtrennen von 0,5 g weiterhin unlöslichen Verunreinigungen vom Fp. 120 bis 300°C erhält man ein Filtrat, das auf die oben beschriebene Weise zunächst bei 70°C, dann bei 4°C mit 5 n Salzsäure angesäuert, gewaschen und getrocknet wird, wobei nochmals 6,5 g N-Benzoyl-chloranthranilsäure (Fp. 208 bis 210°C) anfallen, die Gesamtausbeute beträgt 39,2 g = 71,5 % der berechneten Menge.

Beispiel 3

48 g 3-Trifluormethylbenzoylfluorid und 20 g 50 %ige wäßrige Natronlauge werden innerhalb von 25 min bei 25 bis 36°C unter Rühren gleichzeitig über 2 Zulaufvorrichtungen zu einer Mischung von 42,9 g 6-Chloranthranilsäure, 0,45 g Triethylbenzylammoniumchlorid und 700 g Toluol zugesetzt. Es wird 90 min bei 27°C nachgerührt und dann bei 87 bis 104°C das Wasser ausgekreist. Nach Zugabe von 32,7 g Thionylchlorid während 15 min bei 70°C wird noch 90 min bei 82°C gerührt. Man extrahiert je einmal mit Wasser und mit 0,4 n Natronlauge und engt ein. Man erhält 75 g (=92 %) 5-Chlor-2-(m-trifluormethylphenyl)-4H-3,1-benzoxazin-4-on (Fp. 116 bis 120°C).

Beispiel 4

Ausgehend von 42,9 g 6-Chloranthranilsäure, 39,6 g 3-Fluorbenzoylchlorid, 20 g 50 %iger Natronlauge, 0,45 g Benzyltriphenylphosphoniumchlorid, 620g 1,2-Dichlorethan und 32,7 g Thionylchlorid erhält man nach der in Beispiel 1 beschriebenen Arbeitsweise 68,8 g 5-Chlor-2-(m-fluorphenyl)4H-3,1-benzoxazin-4-on (Fp. 120 bis 123°C; Ausbeute praktisch quantitativ; Reinheit (HPLC): 98 %).

Beispiel 5

Ausgehend von 42,9 g 6-Chloranthranilsäure, 58,4 g 3-Chlordifluormethoxybenzoylfluorid, 20,8 g 50 %iger Natronlauge, 0,45 g Benzyltrimethylammoniumchlorid, 700 g 1,2-Dichlorethan und 32,7 g Thionylchlorid erhält man nach der in Beispiel 1 beschriebenen Arbeitsweise 88,6 g (99 %) 5-Chlor-2-(m-chlordifluormethoxyphenyl)-4H-3,1-benzoxazin-4-on (Fp. 112 bis 114°C).

Beispiel 6

Ausgehend von 38,8 g 6-Fluoranthranilsäure, 39 g 4-Fluorbenzoylchlorid, 20 g 50 %iger Natronlauge, 0,45 g Benzyltrimethylammoniumchlorid, 700 g 1,2-Dichlorethan und 32,7 g Thionylchlorid erhält man nach der in Beispiel 1 beschriebenen Arbeitsweise 62 g (96 %) 5-Fluor-2-(p-fluorphenyl)-4H-3,1-benzoxazin-4-on (Fp. 189 bis 193°C).

Beispiel 7

33,6 g 4-Trifluormethylmercaptobenzoylfluorid und 12 g 50 %ige Natronlauge werden innerhalb 30 min bei 20 bis 39°C gleichzeitig über 2 Zulaufvorrichtungen unter Rühren zu einer Mischung von 25,7 g 6-Chloranthranilsäure, 0,27 g Benzyltrimethylammoniumchlorid und 450 g 1,2-Dichlorethan zugesetzt. Nach 90min Rühren bei 27°C und anschließendem Auskreisen des Wassers werden bei 70°C 21,5 g Phosphoroxychlorid zugesetzt. Nach 3 Stunden Rühren bei 82°C wird auf 30°C abgekühlt und 3mal mit Wasser extrahiert. Nach dem Einengen, zuletzt bei 140°C/2mbar, erhält an 48,8 g (91 %) 5-Chlor-2-(p-trifluormethylmercaptophenyl)-4H-3,1-benzoxazin-4-on (Fp. 135 bis 140°C).

Beispiel 8

92,3g Benzoylchlorid und 52,6 g 50 %ige, wäßrige Natronlauge werden innerhalb 40 Minuten bei 22 bis 38°C unter Rühren parallel über 2 Zulaufvorrichtungen zu einer Suspension von 128,6 g 6-Chloranthranilsäure und 1,35 g Trimethylbenzylammoniumchlorid in 1870 g 1,2-Dichlorethan gegeben. Nach 30 Minuten Rühren bei 32°C werden während 5 Minuten nochmals 16,3 g. Benzoylchlorid und 9,3 g 50 %ige Natronlauge wie oben zugegeben und 55 Minuten bei 25 bis 30°C gerührt. Anschließend wird während 1 1/4 Stunden unter Erhitzen auf Rückfluß das Wasser ausgekreist, bis die organische Phase im Wasserabscheider keine Trübung mehr zeigt. Nach Zugabe von 0,2 g DMF werden unter Rückfluß innerhalb 1 3/4 Stunden 96 g Phosgen unter Rühren eingegast und dann überschüssiges Phosgen während 5 Minuten durch Einblasen von Stickstoff ausgetragen. Nach Abkühlen auf 40°C wird das Reaktionsgemisch dreimal mit Wasser und einmal mit wenig konzentrierter Kochsalzlösung extrahiert. Die organische Phase wird i.V. zuletzt bei 140°C/20 mbar eingeengt, wobei man 190,5 g (98,6 %) 5-Chlor-2-phenyl-4H-3,1-benzoxazin-4-on vom Fp. 153 bis 156°C erhält; die HPLC-Reinheit beträgt 97,4 %.

Vergleichsversuch zu Beispiel 8

40 g 50 %ige, wäßrige Natronlauge werden innerhalb 10 Minuten unter Rühren bei 20 bis 30°C zu einer Suspension von 85,8 g 6-Chloranthranilsäure in 1250 g 1,2-Dichlorethan gegeben. Die Salzsuspension wird unter Erhitzen auf Rückfluß während 1 Stunde am Wasserabscheider vom Wasser befreit. Anschließend werden innerhalb 40 Minuten 56,2 g Benzoylchlorid bei 25 bis 35°C zugegeben und 30 Minuten nachgerührt. Unter gleichen Bedingungen werden nochmals 14,1 g Benzoylchlorid innerhalb 5 Minuten zugegeben und 55 Minuten bei 25°C nachgerührt. Nach Zugabe von 0,2 g DMF werden unter Rückfluß während 1 1/2 Stunden 64 g Phosgen unter Rühren eingegast und dann überschüssiges Phosgen durch Einblasen von Stickstoff ausgetragen. Das Reaktionsgemisch wird auf 40°C abgekühlt, dreimal mit Wasser und einmal mit wenig konzentrierter Kochsalzlösung extrahiert. Nach dem Einengen der organischen Phase, zuletzt bei 140°C/20 mbar werden 115 g (89 %) 5-Chlor-2-phenyl--4H-3,1-benzoxazin-4-on vom Fp. 148 bis 152°C erhalten. Nach der HPLC-Untersuchung und Vergleich mit einem Analysenstandard beträgt der Gehalt 86 %.

Vergleichsversuch zu Beispiel 8

Unter gleichen Reaktionsbedingungen wie in dem vorstehenden Vergleichsversuch, jedoch unter Vorgabe von 0,9 g Trimethylbenzylammoniumchlorid zu der Suspension von 85,8 g 6-Chloranthranilsäure in 1250 g 1,2-Dichlorethan erhält man nach der Aufarbeitung 120,9 g (93,9 %) 5-Chlor-2-phenyl-4H-3,1-benzoxazin-4-on vom Fp. 152 bis 153°C. Der Gehalt beträgt nach der HPLC-Untersuchung 87,5 %.

Beispiel 9

Unter den Reaktionsbedingungen des Beispiels 1 werden 14,4 g m-Trifluormethylbenzoylfluorid, 6 g 50 %ige wäßrige Natronlauge, 11,6 g 5-Fluoranthranilsäure und 0,15 g Trimethylbenzylammoniumchlorid in 185 g 1,2-Dichlorethan miteinander umgesetzt und dann mittels 11 Teilen Thionylchlorid cyclisiert. Nach dem Aufarbeiten erhält man 20,8 g (90 %) 6-Fluor-2-(m-trifluormethylphenyl)-4H-3,1-benzoxazin-4-on vom Fp. 118 bis 119°C.

Beispiel 10

Unter den Reaktionsbedingungen des Beispiels 1, jedoch unter Verwendung von 11,6 g 4-Fluoranthranilsäure und 14,4 g p-Trifluormethylbenzoylfluorid erhält man 21,1 g (91 %) 7-Fluor-2-(p-trifluormethylphenyl)-4H-3,1-benzoxazin-4-on vom Fp. 137 bis 139°C.

Beispiel 11

Unter den Reaktionsbedingungen des Beispiels 1, jedoch unter Verwendung von 11,3 g 5-Methylanthranilsäure und 13,1 g 3-Chlorbenzoylchlorid erhält man 18,5 g (91 %) 6-Methyl-2-(m-chlorphenyl)-4H-3,1-benzoxazin-4-on vom Fp. 160 bis 162°C.

## Ansprüche

1. Verfahren zur Herstellung von 2-Phenyl-4H-3,1-benzoxazin-4-onen der allgemeinen Formel I

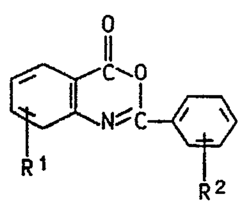

(I),

worin $R^1$ und $R^2$ Wasserstoff oder Halogen, $R^1$ ferner Methyl oder Methoxy, $R^2$ ferner Halogenalkyl, Halogenalkoxy, Halogenalkylmercapto oder Halogenalkylsulfonyl mit jeweils 1 bis 3 Kohlenstoffatomen bedeutet, durch Umsetzung einer entsprechenden Anthranilsäure mit einem entsprechenden Benzoylhalogenid in Gegenwart einer Base - (Acylierung) und Ringschluß durch Wasserentzug - (Cyclisierung), dadurch gekennzeichnet, daß man die Anthranilsäure oder deren Alkalisalz in einem mit wäßriger Alkalilauge nicht mischbaren Suspensionsmittel, gegebenenfalls mit einer jeweils stöchiometrischen Menge mindestens 25 %iger Alkalilauge einerseits und jedenfalls Benzoylhalogenid andererseits gleichzeitig oder nacheinander in Gegenwart einer katalytisch wirksamen Menge eines Phasentransferkatalysators versetzt und die gebildete N-Benzoylanthranilsäure nach Entfernung des Wassers in an sich bekannter Weise cyclisiert.

2. Verfahren nach Anspruch 1, dadurch gekennzeichnet, daß die Acylierung bei einer Temperatur von 10 bis 50°C vorgenommen wird.

3. Verfahren nach einem der Ansprüche 1 und 2, dadurch gekennzeichnet, daß die Cyclisierung mittels Phosgen, Phosphorpentachlorid, Phosphortrichlorid oder Phosphoroxychlorid bei 20 bis 140°C vorgenommen wird, ohne daß die als Zwischenprodukt gebildete N-Benzoylanthranilsäure zuvor isoliert wird.

4. Verfahren nach einem der Ansprüche 1 bis 3, dadurch gekennzeichnet, daß das Wasser azeotrop mit einem Teil des Lösungsmittels aus dem Reaktionsgemisch entfernt wird.